# EUROPEAN PATENT APPLICATION

(11) **EP 2 356 942 A1**
(43) Date of publication of application: **17.08.2011**
(21) Application number: 09827477.2
(22) Date of filing: 02.11.2009
(51) Int. Cl.: A61B 8/08

(54) **ULTRASONOGRAPHIC DEVICE**

(30) Priority: 18.11.2008 JP 2008294434
(71) Applicant: The University of Tokyo, Bunkyo-Ku Tokyo 113-8654 (JP); Aloka Co., Ltd., Mitaka-shi Tokyo 181-8622 (JP)
(72) Inventor: NAKAMURA Kozo, Tokyo 113-8654 (JP); ONISHI Isao, Tokyo 113-8654 (JP); MATSUYAMA Juntaro, Tokyo 113-8654 (JP); TOBITA Kenji, Tokyo 113-8654 (JP); SAKAI Ryoichi, Mitaka-shi Tokyo 181-8622 (JP)
(74) Representative: Heim, Florian Andreas
(86) International application number: PCT/JP2009/068774
(87) International publication number: WO 2010/058696

(57) **Abstract**

An ultrasonic beam (40) for echo tracking is formed for a healthy portion (52a) of a bone (50) containing healthy portions (52a, 52b) and a fractured portion (54). Echo tracking processing is executed over the state where a load F is not applied to the bone (50) and the state where the load F is applied to the bone (50) so as to calculate a distortion amount of the bone (50) accompanying the load F via the ultrasonic beam (40) formed for the healthy portion (52a). The state of union of the fractured portion (54) is evaluated in accordance with the distortion amount obtained via the healthy portion (52a).

## Description

### TECHNICAL FIELD

The present invention relates to an ultrasonic diagnosis apparatus which is used for diagnosis of bones.

### BACKGROUND ART

In order to diagnose bone metabolic diseases such as osteoporosis, to determine the risk of fracture, or to quantitatively diagnose bone union after treatment of bone fracture, simple and quantitative measurements for dynamic properties such as a bone strength are desired.

While assessments of bone formation and bone union depend greatly on X-ray radiography, bone strength is difficult to diagnose by X-ray radiography. While a strength test for a sample bone from a measurement target has been known as a conventional method for measuring bone strength, this method requires an operation of extracting a sample bone, which is invasive. Further, general X-ray CT, DXA (double-energy x-ray absorption), and the like have been put into practical use as methods for measuring bone mass and bone density. However, these techniques, which are essentially means for measuring bone mass, cannot assess bone strength, and are not non-invasive, in that the body is irradiated with X-rays.

Other attempts to quantitatively assess bone strength include known methods including a strain gauge method in which a strain gauge is mounted to an external fixtator for measuring the strain of the fixtator, an oscillation wave method in which oscillations are externally applied to a bone to assess the natural frequency, and an acoustic emission method for detecting acoustic waves generated by a bone which produces a yield stress, or other methods. These methods, however, still suffer from problems of limitations on the treatments to which these methods are adaptable, the methods being invasive to bones, and limited accuracy of assessments.

In view of the background described above, the present inventors have proposed an ultrasonic diagnosis apparatus for non-invasively and quantitatively assessing the dynamic properties of bones. (See Patent Documents 1 to 3.)

[Patent Document 1] JP 2004-298205 A
[Patent Document 2] JP 2006-334273 A
[Patent Document 3] JP 2005-152079 A

### DISCLOSURE OF THE INVENTION

### PROBLEMS TO BE SOLVED BY THE INVENTION

In the ultrasonic diagnosis apparatus described in the above Patent Documents, a plurality of ultrasonic beams are formed with respect to a bone, a plurality of echo signals corresponding to the ultrasonic beams are acquired, with a surface point corresponding to a bone surface being specified for each echo signal, and shape data of the bone surface are generated based on a plurality of surface points obtained by the plurality of echo signals. Then, dynamic properties of the bone are assessed based on a change in the shape data in a case where an external force is applied to the bone. According to the above technique, there can be achieved a revolutionary technology in which the dynamic properties of a bone within a living organism can be assessed non-invasively and quantitatively based on the shape data of the bone surface generated by echo data.

The present inventors have conducted further research and development on improvement of the revolutionary technology described in the above Patent Documents.

The present invention was conceived during the course of their research and development and is aimed at providing an improved ultrasonic diagnosis apparatus which is utilized in diagnosis of a bone including a healthy portion and a diseased portion.

### SOLUTION TO PROBLEMS

In order to attain the above object, an ultrasonic diagnosis apparatus according to a preferable aspect of the invention includes a transmission/reception section for forming a plurality of ultrasonic beams with respect to a healthy portion of a bone including the healthy portion and a diseased portion, a surface tracking section for detecting a surface point corresponding to a bone surface for each of the ultrasonic beams and tracking a plurality of surface points corresponding to the plurality of ultrasonic beams, a property amount calculating section for calculating a property amount reflecting a dynamic property of the bone based on a plurality of surface points which are displaced by application of an external force to the bone, and a recovery state assessing section for assessing a recovery state of the diseased portion based on the property amount which is obtained via the healthy portion.

In the above aspect, a healthy portion of a bone refers to a portion of the bone which is relatively healthy, and a diseased portion of a bone refers to a portion of the bone which is relatively diseased. The diseased portion of the bone encompasses a fractured portion, a cracked portion, or the like, and also encompasses a portion in which union of fraction or crack is in progress or a completely recovered portion.

According to the above aspect, the recovery state of a diseased portion can be assessed based on the amount of a property which can be obtained through a healthy portion. With this structure, as the property amount that can be obtained through a healthy portion in which the reflection state of ultrasound is relatively good, for example, the accuracy of assessment and reproducibility of the recovery state of a diseased portion can be increased as compared to the case where the property amount is obtained through a bone portion in which the reflection state of ultrasound is unstable. Further, because the position of the ultrasonic beam is not limited to the diseased portion, the degree of freedom for measurements for assessing the recovery state of the diseased portion can be increased.

In accordance with a preferable aspect, the property amount calculating section calculates a property amount reflecting distortion of the bone, based on a plurality of surface points which are displaced by application of a load to the bone.

In accordance with a preferable aspect, the recovery state assessing section forms time-varying change data indicating a change with time of the property amount obtained via the healthy portion.

In accordance with a preferable aspect, a state of progress of bone union in a fractured portion which is the diseased portion is assessed based on the time-varying change data.

In accordance with a preferable aspect, the state of progress of bone union in the fractured portion is assessed from a comparison between a reference value of a property amount corresponding to distortion of a healthy bone and the time-varying change data.

Also, in order to attain the above object, an ultrasonic diagnosis apparatus according to a preferable aspect of the present invention includes a transmission/reception section for forming a plurality of ultrasonic beams with respect to a healthy portion of a bone including the healthy portion and a diseased portion, a surface tracking section for detecting a surface point corresponding to a bone surface for each of the ultrasonic beams and tracking a plurality of surface points corresponding to the plurality of ultrasonic beams, and a property amount calculating section for calculating a property amount reflecting a dynamic property of the bone based on a plurality of surface points which are displaced by application of an external force to the bone, and the ultrasonic diagnosis apparatus is used for assessing a recovery state of the diseased portion based on the property amount which is obtained via the healthy portion.

### ADVANTAGEOUS EFFECTS OF INVENTION

According to the present invention, there is provided an improved ultrasonic diagnosis apparatus which is utilized for diagnosis of a bone including a healthy portion and a diseased portion. For example, in accordance with a preferable aspect, it is possible to assess the recovery state of the diseased portion based on the property amount obtained through the healthy portion.

### BRIEF DESCRIPTION OF THE DRAWINGS

FIG. 1 is a block diagram illustrating the overall structure of an ultrasonic diagnosis apparatus according to the present invention;
FIG. 2 is a view for explaining how tracking of a bone surface portion is performed;
FIG. 3 is a view for explaining a method of calculating the distortion amount of a bone;
FIG. 4 is a view for explaining an example of bone diagnosis according to the present embodiment;
FIG. 5 is a view for explaining an example of assessment of a bone union state according to the present embodiment;
FIG. 6 is a view for explaining a modification example of bone diagnosis according to the present embodiment; and
FIG. 7 is a view illustrating a probe capable of applying a load F to the bone.

### REFERENCE SYMBOLS LIST

10 probe, 12 transmission/reception section, 22 surface shape measuring section, 24 property amount calculating section, 26 union state assessing section

### BEST MODE FOR CARRYING OUT THE INVENTION

A preferred embodiment of the present invention will now be described.

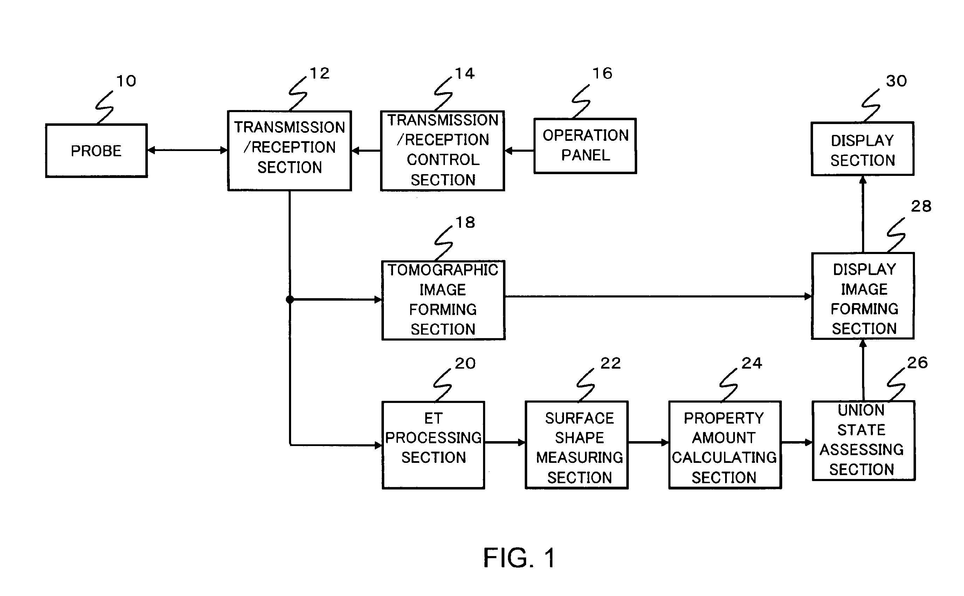

FIG. 1 illustrates a preferable embodiment of an ultrasonic diagnosis apparatus according to the present invention, and is a block diagram illustrating the overall structure of the ultrasonic diagnosis apparatus. A probe 10 is preferably an ultrasonic probe which is used in contact with a body surface of a subject. As a matter of course, an ultrasonic probe which is inserted into a subject for use may also be used. The probe 10 forms an ultrasonic beam directed toward a bone within the body of the subject. As the probe 10, while a linear electronic scan probe (a linear probe) for electronically scanning an ultrasonic beam is preferable, other methods such as sector electronic scanning may be used. While the bone to be diagnosed may be, for example, the tibia, the fibula, or the like, the subject of diagnosis for the ultrasonic diagnosis apparatus according to the present invention is not limited to these bones.

A transmission/reception section 12 controls the probe 10 to electronically scan an ultrasonic wave within a cross sectional plane. If the probe 10 is a linear probe, 120 ultrasound beams, for example, are sequentially scanned, and an echo signal is acquired for each of the ultrasonic beams. A plurality of echo signals which are acquired are output to a tomographic image forming section 18, which then forms a tomographic image (B mode image) of a bone based on the plurality of echo signals. The B mode image thus formed is displayed on a display section 30 via a display image forming section 28.

The echo signals acquired in the transmission/reception section 12 are also output to an echo tracking processing section (ET processing section) 20. The echo tracking processing section 20 performs so-called echo tracking processing by extracting a bone surface portion from each echo signal for tracking. For example, the technology which is described in detail in JP 2001-309918 A is used, the summary of which is as follows.

The echo signal acquired by the probe 10 has a large amplitude in a portion corresponding to the bone surface. If the bone surface portion is simply interpreted as a portion with a large amplitude, it is not clear which portion in the scope of large amplitudes corresponds to the surface portion, and this results in generation of an extraction error which approximately corresponds to the scope of large amplitudes (which is approximately 0.2 mm in a general ultrasonic diagnosis apparatus). With the echo tracking processing, the extraction accuracy is drastically improved (the extraction accuracy can be improved to approximately 0.002 mm) by detecting a zero crossing point as a representative point of an echo signal and tracking the detected zero crossing point. The zero crossing point is detected at a timing of inversion of the amplitude of the echo signal from positive to negative or at a timing of inversion of the polarity of the echo signal from negative to positive during a tracking gate period. Upon detection of a zero crossing point, a new tracking gate is set while the point is used as a center. Then, concerning an echo signal to be acquired at the following timing, a zero crossing point is detected during the newly set tracking gate period. In this manner, for each ultrasonic beam, a zero crossing point of an echo signal is tracked as a surface point, so that the position of the bone surface can be measured with a high degree of accuracy using the probe 10 as a reference.

For the echo tracking processing, five tracking echo signals, for example, are used. The tracking echo signals may be selected from among echo signals for use in tomographic image forming (e.g. 120 echo signals). Alternatively, five tracking echo signals may be formed separately from the beams for tomographic image forming.

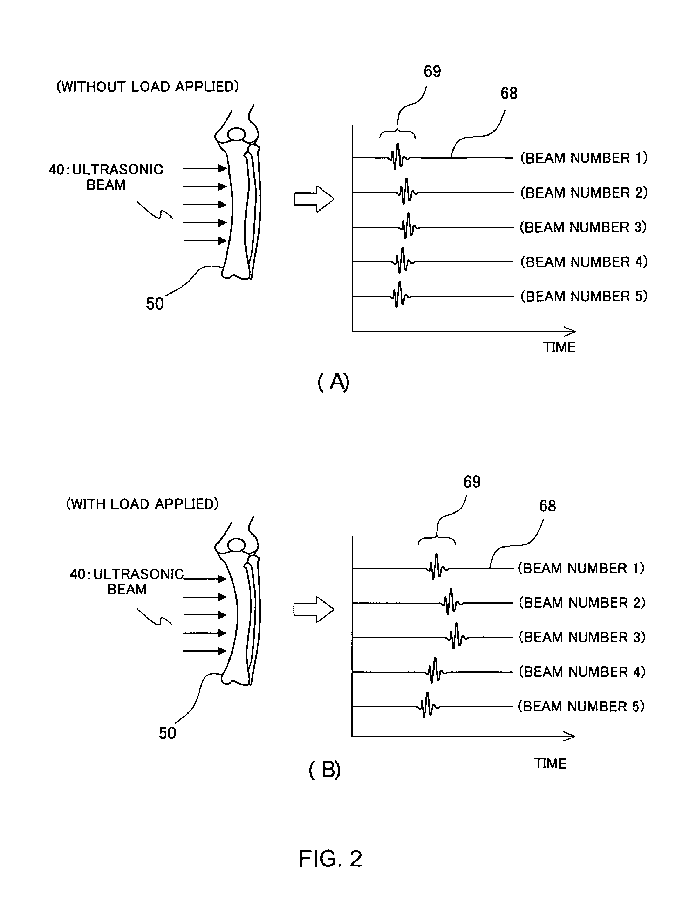

FIG. 2 is a view for explaining how tracking of the bone surface portion by five echo signals is performed. In the assessment of dynamic properties of a bone using the ultrasonic diagnosis apparatus according to the present invention, a displacement of the surface point between a state in which a load is not applied to a bone (non-loaded state) and a state in which a load is applied to the bone (loaded state) is measured for each echo signal (for each ultrasonic beam).

FIG. 2(A) illustrates tracking with respect to a bone 50 in a non-loaded state. Each echo signal 68 corresponding to five respective ultrasonic beams 40 directed to the bone 50 exhibits a large amplitude (an amplitude maximum portion 69) at a portion corresponding to the bone surface. Beam numbers 1 to 5 are sequentially assigned to the five ultrasonic beams 40 from top to bottom in the figure. As the zero crossing point is detected as a surface point within the amplitude maximum portion 69 for each ultrasonic beam, the position of the surface point can be specified with a very high level of precision.

FIG. 2(B) illustrates tracking with respect to the bone 50 in a loaded state. As in the case of FIG. 2(A), it is possible to recognize the shape of the bone surface based on the echo signals 68 corresponding to the five respective ultrasonic beams 40. Here, due to application of a load, the strain of the bone 50 (the degree of bone deflection) in FIG. 2(B) is larger than that of the bone 50 in FIG. 2(A). While an example with five tracking echo signals has been described with reference to FIG. 2, a plural number of echo signals other than five echo signals can be measured.

Referring back to FIG. 1, a surface shape measuring section 22 measures the displacement of the surface point (tracking point) between the non-loaded state and the loaded state, to measure the displacement of the surface point from each of the five ultrasonic beams. More specifically, with regard to each of the ultrasonic beams 40 assigned the beam numbers 1 to 5 illustrated in FIG. 2, the moving amount of the zero crossing point between the non-loaded state and the loaded state is measured. As a result, the displacements concerning five points on the bone surface are obtained.

Further, the displacements concerning the five points on the bone surface are reflected as actual measured points on a coordinate system formed by an axis indicating the beam positions of the ultrasonic beam 40 and an axis indicating the displacement of the surface points. Then, an interpolation line connecting a plurality of actual measured points is generated, and based on the degree of curvature of this interpolation line, a distortion amount of the bone is calculated as a property amount for assessing the dynamic property of the bone by a property amount calculation section 24.

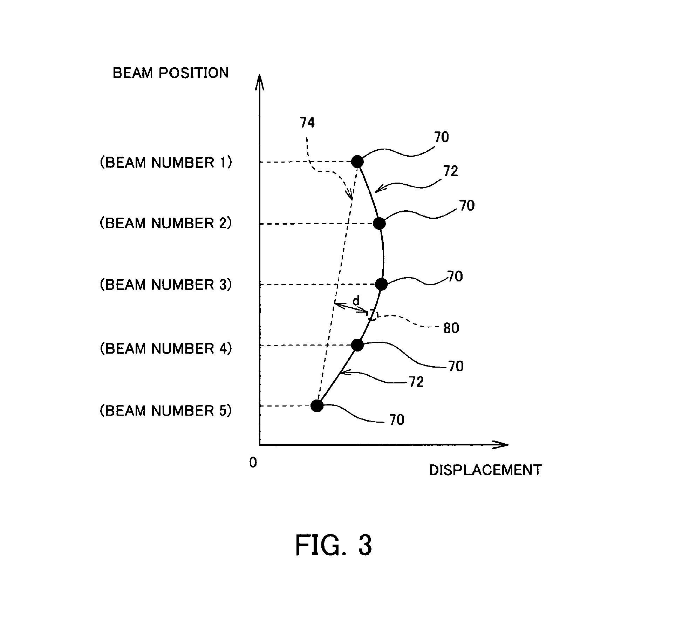

FIG. 3 is a view for explaining a method of calculating the distortion amount of a bone. FIG. 3 illustrates a coordinate system in which the vertical axis indicates the beam position of an ultrasonic beam and the horizontal axis indicates a displacement of a surface point. On this coordinate system, five actual measured points 70 are reflected as points correlating the beam position of the respective ultrasonic beams and the displacement measured with the ultrasonic beams. The beam positions correspond to the positions of the respective ultrasonic beams for echo tracking (the positions of the ultrasonic beams 40 in FIG. 2), and are specified by the beam numbers 1 to 5 sequentially from the top of FIG. 3.

Further, the surface shape measuring section 22 (FIG. 1) generates an interpolation line 72 connecting the five actual measured points 70. The interpolation line 72 can be obtained by applying curve interpolation to the five actual measured points 70 by using spline interpolation, least squares interpolation, or the like.

In addition, the property amount calculating section 24 (FIG. 1) assesses the degree of curvature of the interpolation line 72 based on a comparison between a straight line 74 connecting two end points among the five actual measured points 70 and the interpolation line 72. More specifically, based on a distance d between a maximum displacement point 80 existing on the interpolation line 72 at a position which is furthest from the straight line 74 and the straight line 74, ε=d/L is calculated as a distortion amount of the bone from the length L of the straight line 74 and the distance d, for example. Here, the distortion amount ε is referred to as a strain. The property amount calculating section 24 may provide a measurement result in which the strain ε is correlated to the load value at that time.

In the calculation method for the distortion amount which has been described with reference to FIG. 3, the straight line 74 connecting the two end points among the five actual measured points 70 is used. In place of this method, the distortion amount of a bone may be calculated based on a comparison between the interpolation line under the non-loaded state and the interpolation line under the loaded state.

Referring back to FIG. 1, once the distortion amount of the bone is calculated by the property amount calculating section 24 as a property amount of the bone for assessing the dynamic properties of the bone, a union state assessing section 26 assesses the union state of the bone based on the distortion amount of the bone. According to the present embodiment, a bone including a fractured portion and a healthy portion is a target of diagnosis, and the union state of the bone in the fractured portion is assessed based on the distortion amount which is obtained through the healthy portion. The diagnosis of a bone and assessment of the union state will be described below. In the following description, concerning the elements (structures) in FIG. 1 already described, the reference numerals in FIG. 1 will be used.

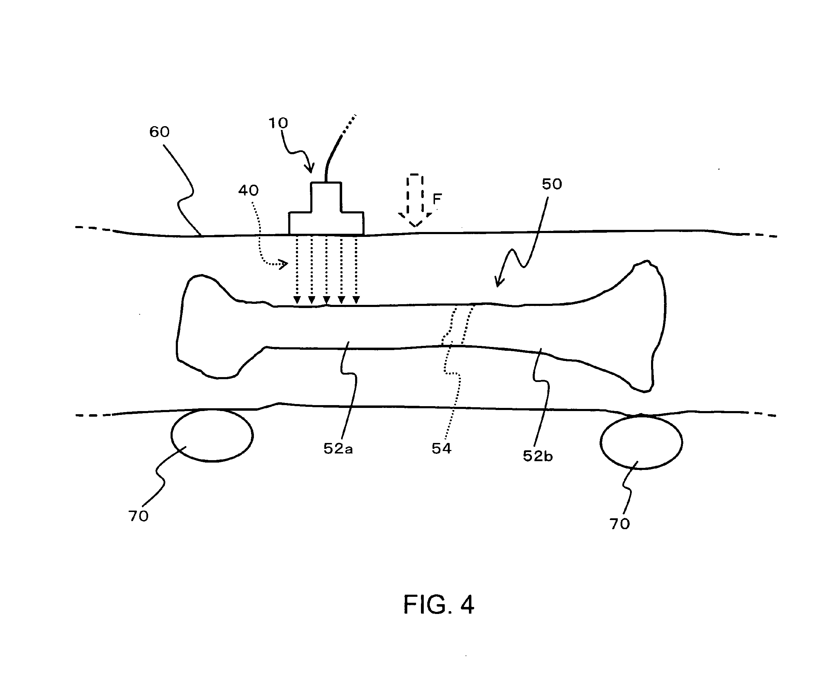

FIG. 4 is a view for explaining an example diagnosis of a bone according to the present embodiment. A bone 50 which is a target of diagnosis is present within a subject body 60 and includes healthy portions 52a and 52b and a fractured portion 54. The probe 10 is used in contact with a body surface of the subject body 60 and forms ultrasonic beams 40 for echo tracking with respect to the healthy portion 52a of the bone 50.

The operator confirms positions of the healthy portions 52a, 52b and the fractured portion 54 by using a B mode image including the bone 50, for example, to adjust the contact position or the like of the probe 10 so as to form five ultrasonic beams 40, for example, with respect to the healthy portion 52a.

FIG. 4 illustrates a three-point bending method in which a load F is applied between two fixed points. More specifically, the rod-like bone 50 is supported from the bottom side in the figure at two points (two fixed points) by two fixing elements 70 contacting the body surface of the subject body 60, and a load F is applied from above in the figure between these two fixed points.

Then, echo tracking processing is performed from a state in which the load F is not applied to the bone 50 through a state in which the load F is applied to the bone 50 (see FIG. 2), and the distortion amount of the bone 50 associated with the load F is calculated via the ultrasonic beams 40 formed with respect to the healthy portion 52a (see FIG. 3).

As described above, according to the present embodiment, a plurality of ultrasonic beams 40 are formed with respect to the healthy portion 52a of the bone 50 including the healthy portions 52a and 52b and the fractured portion 54, and the distortion amount associated with the load F is obtained via the healthy portion 52a.

When the load F is applied to the bone 50, in the example illustrated in FIG. 4, an internal stress to deflect in an arc shape with the two fixed points serving as supporting points acts on the bone 50. In the case of a healthy bone including no fractured points 54 or the like, such an internal stress is generated relatively uniformly over the healthy bone as a whole, so that the deflection (distortion) is also relatively uniform over the healthy bone as a whole.

On the other hand, in the bone 50 including the healthy portions 52a and 52b having relatively high rigidity and the fractured portion 54 having relatively low rigidity, the internal stress associated with the load F tends to act intensively on the fractured portion 54. Accordingly, as compared to the deflection (distortion amount) in the healthy bone, the deflection in the fractured portion 54 is greater and the deflection in the healthy portions 52a and 52b is smaller. Also, as the union in the fractured portion 54 progresses and the state of the bone approaches that of a healthy bone, the deflection in the healthy portions 52a and 52b approaches the deflection of a healthy bone. The present embodiment utilizes such a phenomenon to assess the state of union in the fractured portion 54 based on the distortion amount obtained through the healthy portion 52a.

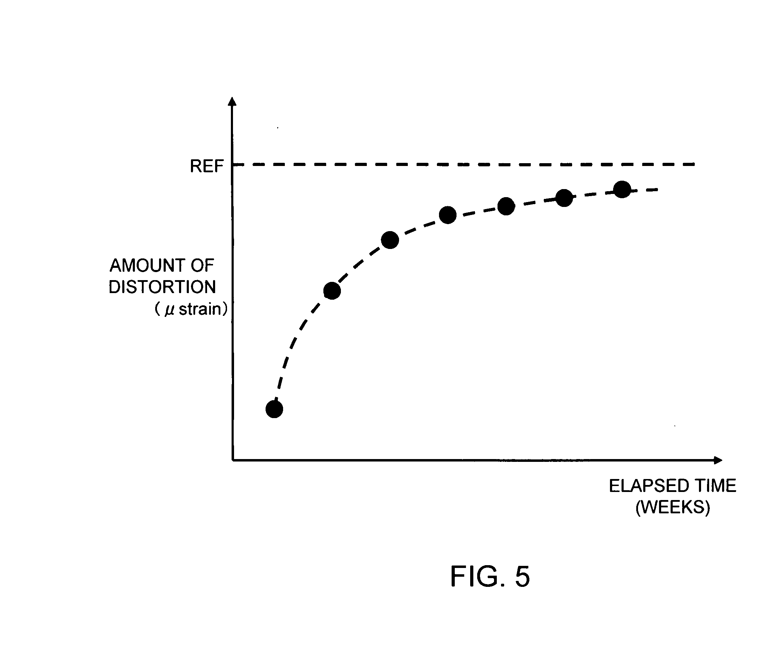

FIG. 5 is a view for explaining an example assessment of a union state of the bone according to the present embodiment. FIG. 5 illustrates one example of time-varying change data indicating a change with time in the distortion amount obtained through a healthy portion. In the time-varying change data (graph) illustrated in FIG. 5, the horizontal axis indicates an elapsed time for bone union, and the vertical axis indicates a distortion amount for each elapsed time. For example, the distortion amount associated with a load is measured through a healthy portion every week (see FIG. 4), and the measurement results over a plurality of weeks are indicated in the sequence of time along the horizontal axis. In FIG. 5, the unit of the distortion amount is micro strain (µ strain).

As illustrated in FIG. 5, as the union of the fractured portion progresses with elapsed time; i.e., with the progress toward the increasing direction (positive direction) along the horizontal axis, the distortion amount which can be obtained through the healthy portion gradually increases. More specifically, the internal stress associated with the load is intensively applied to the fractured portion with the distortion amount in the healthy portion being small in the initial state of the bone union, and as the bone union progresses, the internal stress is dispersed so that the distortion amount in the healthy portion increases.

Accordingly, it is possible, for example, to measure, in advance, a reference value REF of the distortion amount of a healthy bone including no fractured portions or the like and compare the trend of increase in the distortion amount in the healthy portion with the reference value REF, to thereby assess the degree of progress of the bone union. The time-varying change data (graph) illustrated in FIG. 5 is formed in a union state assessing section 26 and is displayed on a display section 30 via a display image forming section 28.

With the assessment of bone union according to the present embodiment, as it is only necessary to induce a stress distribution (a difference in stress) between the fractured portion and the healthy portion, various modification examples can be provided, in addition to the diagnosis example described with reference to FIG. 4.

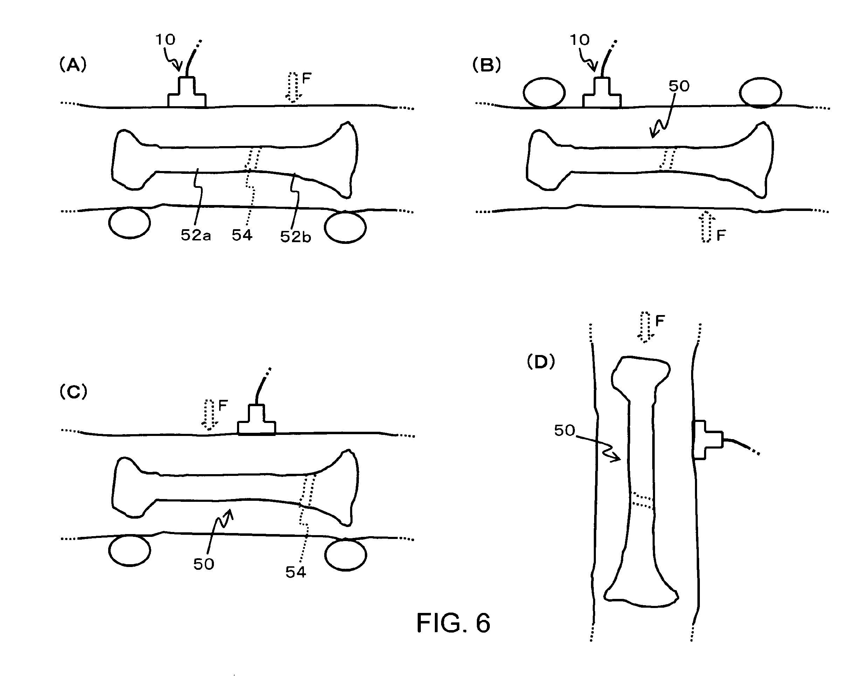

FIG. 6 is a view for explaining modification examples of bone diagnosis according to the present embodiment. FIGs. 6(A) to 6(C) illustrate modification examples of the three-point bending type which has been described above with reference to FIG. 4. For example, as illustrated in FIG. 6(A), it is possible to place the probe 10 on the side of one of the healthy portions sandwiching the fractured portion 54; i.e., on the side of the healthy portion 52a, and apply the load F on the side of the other healthy portion 52b. Further, as illustrated in FIG. 6(B), for example, it is also possible to place the probe 10 on one of the two sides sandwiching the bone 50 therebetween; i.e., the upper side in the drawing, and apply the load F from the other side (lower side in the drawing). In addition, as illustrated in FIG. 6(C), even when the fractured portion is located at the end portion of the bone 50, the assessment for bone union according to the present embodiment can be used. Here, in place of the three-point bending method described with reference to FIG. 4, the load F may be applied along the major axis direction of the bone 50 as illustrated in FIG. 6(D). Further, the load F may be applied to the bone via the probe 10.

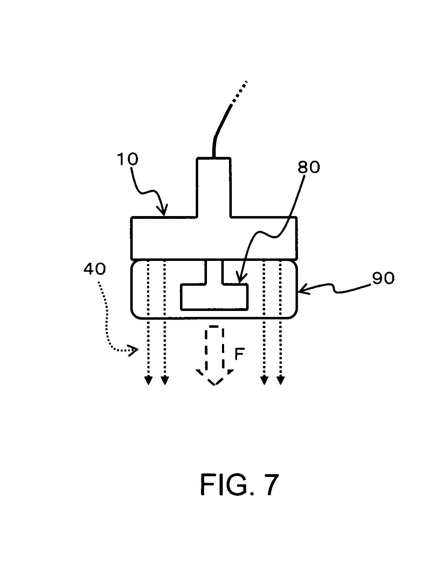

FIG. 7 is a view illustrating a probe 10 capable of applying a load F to the bone. The probe 10 illustrated in FIG. 7 includes a compressor 80 on a transducer surface thereof. Further, the probe 10 in FIG. 7 includes a water bag 90 which covers the whole transducer surface so as to enclose the compressor 80. The interior of the water bag 90 is filled with a medium (e.g. water) which is suitable for propagation of ultrasound waves.

By bringing the probe 10 illustrated in FIG. 7 into contact with the body surface of the subject, the load F is applied to the bone within the subject body through the compressor 80. Further, ultrasonic beams 40 are formed with respect to the bone within the subject by transmission and reception of ultrasound via the medium within the water bag 90.

A preferred embodiment of the present invention has been described above. According to the embodiment described above, as the distortion amount can be obtained via the healthy portion in which the reflection state of the ultrasound is relatively good, the precision of assessment and reproducibility for the union state of the fractured portion can be increased as compared to a case in which the distortion amount is obtained via the fractured portion in which the reflection state of the ultrasound is unstable. In the present embodiment, as the assessment can be achieved with a single probe, the workability for measurement can be increased. Also, as the positions of the ultrasonic beams, the load, and the fixed points are not limited to, for example, the fractured portion and the area near the fractured portion, the degree of freedom of the measurement can also be increased. The position of the probe may be corresponded to the position of the load, or to the position of the fixed point.

The embodiment and the advantages thereof described above are only mere examples in all respects, and do not limit the scope of the present invention. For example, as a property amount reflecting the dynamic property of the bone, the property amount corresponding to viscoelasticity (e.g., the hysteresis property between the load value and the distortion amount described in FIG. 12 of Patent Document 1) or a property amount corresponding to plastic deformation of the bone (e.g. the plastic deformation component described in FIG. 4 of Patent Document 3) may be used in place of the distortion amount of the bone. Further, a mode in which the union state assessing section 26 which has been described with reference to FIG. 1 is not provided within the ultrasonic diagnosis apparatus is also possible. In this case, it is possible, for example, to implement the functions of the union state assessing section 26 in a computer and assess the degree of progress of bone union or the like by means of an ultrasonic system achieved by a combination of a computer and an ultrasonic diagnosis apparatus. As such, the present invention includes a variety of modes within a scope not departing from the essence of the present invention.

## Claims

1. An ultrasonic diagnosis apparatus comprising:
a transmission/reception section for forming a plurality of ultrasonic beams with respect to a healthy portion of a bone including the healthy portion and a diseased portion;
a surface tracking section for detecting a surface point corresponding to a bone surface for each of the ultrasonic beams and tracking a plurality of surface points corresponding to the plurality of ultrasonic beams;
a property amount calculating section for calculating a property amount reflecting a dynamic property of the bone based on a plurality of surface points which are displaced by application of an external force to the bone; and
a recovery state assessing section for assessing a recovery state of the diseased portion based on the property amount which is obtained via the healthy portion.

2. The ultrasonic diagnosis apparatus according to Claim 1, wherein
the property amount calculating section calculates a property amount reflecting distortion of the bone, based on a plurality of surface points which are displaced by application of a load to the bone.

3. The ultrasonic diagnosis apparatus according to Claim 2, wherein
the recovery state assessing section forms time-varying change data indicating a change with time of the property amount obtained via the healthy portion.

4. The ultrasonic diagnosis apparatus according to Claim 3, wherein
a state of progress of bone union in a fractured portion which is the diseased portion is assessed based on the time-varying change data.

5. The ultrasonic diagnosis apparatus according to Claim 4, wherein
the state of progress of bone union in the fractured portion is assessed from a comparison between a reference value of a property amount corresponding to distortion of a healthy bone and the time-varying change data.

6. An ultrasonic diagnosis apparatus comprising:
a transmission/reception section for forming a plurality of ultrasonic beams with respect to a healthy portion of a bone including the healthy portion and a diseased portion;
a surface tracking section for detecting a surface point corresponding to a bone surface for each of the ultrasonic beams and tracking a plurality of surface points corresponding to the plurality of ultrasonic beams; and
a property amount calculating section for calculating a property amount reflecting a dynamic property of the bone based on a plurality of surface points which are displaced by application of an external force to the bone,
wherein the ultrasonic diagnosis apparatus is used for assessing a recovery state of the diseased portion based on the property amount which is obtained via the healthy portion.
